# EUROPEAN PATENT APPLICATION

(11) **EP 1 421 954 A1**
(43) Date of publication of application: **26.05.2004**
(21) Application number: 02760639.1
(22) Date of filing: 14.08.2002
(51) Int. Cl.: A61K 47/04, A61K 9/48, A61K 47/36, A61K 47/42, A61K 47/46, A61K 47/48, A61K 38/18

(54) **MATERIALS SUSTAINEDLY RELEASING DRUG IN VIVO**

(30) Priority: 30.08.2001 JP 2001262476
(71) Applicant: Japan Science and Technology Agency, Kawaguchi-shi, Saitama 332-0012 (JP); National Institute for Materials Science, Tsukuba-shi, Ibaraki 305-0047 (JP); National Institute of Advanced Industrial Science and Technology, Tokyo 100-0013 (JP)
(72) Inventor: SOTOME, Shinichi, Minato-ku, Tokyo 108-0023 (JP); UEMURA, Toshimasa, Tsukuba-shi, Ibaraki 305-0044 (JP); KIKUCHI, Masanori, Tsukuba-shi, Ibaraki 305-0032 (JP); SHINOMIYA, Kenichi, Kita-ku, Tokyo 114-0014 (JP); TANAKA, Junzo, Tsukuba-shi, Ibaraki 300-3254 (JP)
(74) Representative: Wagner, Karl H., Dipl.-Ing.
(86) International application number: PCT/JP2002/008282
(87) International publication number: WO 2003/020316

(57) **Abstract**

Disclosed is a material for sustained chemical-release in vivo, which comprises a hydroxyapatite/collagen (HAp/Col) nanocomposite formed as a coprecipitate, and a chemical consisting at least one of a drug and a bioactive substance including a growth factor and a DNA enzyme. The hydroxyapatite (HAp) has a specific surface area in the range of 50 to 300 m²/g. The hydroxyapatite (HAp) and the collagen (Col) are combined in such a manner that the crystals of the hydroxyapatite (HAp) are oriented in the C-axis direction around the fibers of the collagen (Col). The chemical is carried on the surface of the hydroxyapatite (HAp), the surface of the collagen (Col) and in a hydrated water of the hydroxyapatite (HAp) and the collagen (Col). The sustained chemical-release material can be prepared in the form of a bioabsorbable capsule, or in the form of allowing in vivo administration by injection and in-vivo gelation after said administration.

## Description

### TECHNICAL FIELD

The present invention relates to a material for sustained chemical-release in vivo, comprising a hydroxyapatite/collagen (HAp/Col) nanocomposite.

### BACKGROUND ART

In conventional sustained drug-release systems, the following drug carriers have been typically used. (1) A material having a high water-storage capacity, such as collagen, is used to carry a drug thereon. When the material with the drug is introduced in vivo, the drug is diffusedly released. (2) A biodegradable biomaterial, such as polylactic acid, is used, and mixed with a drug in the production process thereof. The drug is released in conjunction with in-vivo degradation of the biomaterial.

In relation to the above (1), there has been known a collagen-based product designed for administration by injection and long-term sustained release (Japanese Patent Publication Nos. 05-12328, 05-71566 and 06-94418, and Japanese Patent Laid-Open Publication No. 08-34747).

There has also been known a collagen/calcium phosphate composite material for use as a film for periodontal guided tissue regeneration (GTR), a hemostatic agent, a bone filler material, a cartilage filler material and a substrate for 3-dimensional culture of hard tissue cells (Japanese Patent Laid-Open Publication No. 06-304242). This composite material is obtained by causing a reaction between calcium phosphate compound and collagen in a calcium phosphate solution to form a complex thereof, and drying the complex.

Further, a method of preparing a composite material with a high degree of orientation and for use as artificial bones etc. has been known (Japanese Patent Laid-Open Publication No. 11-199209). The method comprises coprecipitating calcium phosphate and collagen, and then pressing the obtained coprecipitate to form the composite material.

On an experimental basis, collagen has been used as a carrier for various cytokines, growth factors and others in view of its high water-storage capacity or capability of being introduced in vivo while carrying a volume of drug solution. However, a growth-factor carrier using collagen or a hydrous gel substance, such as gelatin, releases a drug therefrom at a high rate after introduction, and thereby can sustain the effect of the drug only in a relatively short period of time. Thus, the carrier consisting of such a martial without modification involves a requirement of using a large volume of growth factor.

Hydroxyapatite is widely used as a biomaterial having high bone-inducing ability, and a number of attempts are being made to use the bone filler material as a carrier for growth factors. It is also known that hydroxyapatite can be attached onto the crystal surface of protein or DNA because of its crystal structure having a significantly strong polarity and a high electrical affinity to protein and DNA. In the field of biochemistry, a nanocrystalline hydroxyapatite has been used as a material for chromatography.

Nanocrystal as used in chromatography is in the form of power practically unavailable for biomaterials, and thus a processed powder material, such as a sintered material, is used as biomaterials. However, such a material inevitably has a significantly reduced surface area for adsorbing a drug, and practically contributes little to a carrier for drugs.

### DISCLOSURE OF INVENTION

In view of the above circumstances, it is therefore an object to provide a material for sustained chemical-release in vivo, capable of carrying a sufficient amount of various kinds of chemical such as drugs, growth factors or DNA enzymes, and releasing the chemical locally at a desired release rate in vivo.

A HAp/Col composite of the present invention comprises nanocrystalline hydroxyapatite and collagen. The HAp/Col composite can carry a volume of growth factor, because it includes hydroxyapatite having a significantly large specific surface area effective to adsorb a growth factor, specifically in the range of about 50 to 300 m²/g, and collagen having high water-storage capacity.

The hydroxyapatite with a large specific surface area is strongly bonded with a growth factor so that the release rate of the growth factor can be reduced to sustain the growth effect in vivo locally and over the long term.

The HAp/Col composite has a high surface-adsorption capacity of growth factors and any other substance having an electrical polarity, such as drugs, and DNA. Thus, the HAp/Col composite can be used as a sustained-release carrier.

Specifically, in order to achieve the above object, the present invention provides a material for sustained chemical-release in vivo, comprising a hydroxyapatite/collagen (HAp/Col) nanocomposite formed as a coprecipitate through a reaction of a phosphoric acid solution, a potassium hydroxide solution and a collagen solution under a given condition, and a chemical consisting at least one of a drug and a bioactive substance including a growth factor and a DNA enzyme. The hydroxyapatite (HAp) has a specific surface area in the range of 50 to 300 m²/g. The hydroxyapatite (HAp) and the collagen (Col) are combined in such a manner that the crystals of the hydroxyapatite (HAp) are oriented in the C-axis direction around the fibers of the collagen (Col). The chemical is carried on the surface of the hydroxyapatite (HAp), the surface of the collagen (Col) and in a hydrated water of the hydroxyapatite (HAp) and the collagen (Col).

The above material may further include glycosaminoglycan (hyaluronic acid, chondroitin sulfate, keratan sulfate, etc.) or hydrogel which has high hydration ability. The glycosaminoglycan or hydrogel is combined with the HAp/Col nanocomposite so that the HAp/Col nanocomposite serves as a bioabsorbable capsule capable of enclosing the growth factor therein.

Alternatively, the above material may be prepared in the form of allowing in-vivo administration by injection and in-vivo gelation after the administration.

### BEST MODE FOR CARRYING OUT THE INVENTION

The HAp/Col composite of the present invention is a biomaterial obtained by combining hydroxyapatite with collagen. The HAp/Col composite is formed by dropping a suspension of potassium hydroxide and a phosphoric acid solution containing collagen simultaneously into a distilled water to form a coprecipitate, and pressure-dehydrating the coprecipitate.

Through this process, the HAp/Col composite is formed as a nanocomposite in which HAp and Col are combined in such a manner that HAp crystals are oriented in the C-axis direction around Col fibers. The hydroxyapatite has a significantly large specific surface area in the range of about 50 to 300 m²/g, which is measured by a BET method. The crystal of the hydroxyapatite contained in the HAp/Col composite is small in size (equal to or less than that of the hydroxyapatite for use in chromatography). Thus, the hydroxyapatite can effectively adsorb a volume of the chemical on the surface thereof, and can additionally draw and hold the chemical contained in the collagen.

The combination of the collagen having a high water-storage capacity and the hydroxyapatite having a high affinity to a chemical allows the release rate of the chemical to be reduced so as to release the chemical such as a growth factor locally and sustainedly over a long term. That is, in the sustained drug-release material of the present invention, two types of materials are combined to mutually compensate for their disadvantages as a chemical carrier and mutually bring out their advantages.

### [EXAMPLE] Use as FGF 2 (Fibroblast Growth Factor) Carrier

A plurality of hydroxyapatite/collagen composite (hereinafter referred to as "HAp/Col") plates having a disc shape of 12 mm diameter × 0.5 mm thickness were prepared. The HAp/Col plates were immersed in a phosphoric acid buffer solution, and left at 37°C for 24 hours. Then, the HAp/Col plates were divided into a FGF 2 (-) group and a FGF 2 (+) group, and the groups were immersed in the above buffer solution at 4°C for 72 hours.

Then, the FGF 2 (-) group was immersed in 5 ml of Tyrode solution (a buffer solution for checking platelet-associated immune globulin). The FGF 2 (+) group was immersed in a mixture of 5 ml of Tyrode solution and b FGF (20 ng/ml). The HAp/Col plates were taken out of the respective solutions, and rinsed out with a pure Tyrode solution 3 times to remove FGF 2 residing on the surfaces thereof (the 3rd rinsing was performed for 45 minutes).

Then, the HAp/Col plates were placed on a 6-hole culture plate, and a cell-suspending liquid (5 × 10³ cells/ml) of the culture plate was seeded to the HAp/Col plates. After 24 hours, the HAp/Col plates were moved to culture vessels (one plate per vessel) of 10 cm diameter, and the cells in each of the HAp/Col plates were cultured in 20 ml of culture medium. Subsequently, the culture medium was renewed every 2 days. After 5 days from the seeding of the cells, the cultured cells were stained with propyliodine, and observed by a confocal microscope. As a result, almost no cell proliferation was observed in the FGF 2 (-) group containing no growth factor.

By contrast, an active cell proliferation was observed in the FGF 2 (+) group carrying the growth factor. This result verifies that the composite of the present invention exhibits an excellent characteristic as a sustained release carrier for FGF 2.

### INDUSTRIAL APPLICABILITY

The sustained chemical-release material of the present invention is usable in the field of tissue engineering as a scaffold material in tissue regeneration, which provides an area capable of carrying a growth factor for controlling the proliferation/differentiation of cells, and proliferating cells. Further, in the field of regeneration medicine, the sustained chemical-release material can be used as a bone filler material containing a growth factor, a drug, etc. for promoting bone regeneration.

## Claims

1. A material for sustained chemical-release in vivo, comprising:
a hydroxyapatite/collagen (HAp/Col) nanocomposite formed as a coprecipitate; and
a chemical consisting at least one of a drug and a bioactive substance including a growth factor and a DNA enzyme,
wherein said hydroxyapatite (HAp) has a specific surface area in the range of 50 to 300 m²/g,
said hydroxyapatite (HAp) and said collagen (Col) are combined in such a manner that the crystals of said hydroxyapatite (HAp) are oriented in the C-axis direction around the fibers of said collagen (Col), and
said chemical is carried on the surface of said hydroxyapatite (HAp), the surface of said collagen (Col) and in a hydrated water of said hydroxyapatite (HAp) and said collagen (Col).

2. The material as defined in claim 1, which further includes glycosaminoglycan or hydrogel which has high hydration ability, wherein said glycosaminoglycan or hydrogel is combined with said HAp/Col nanocomposite so that said HAp/Col nanocomposite serves as a bioabsorbable capsule capable of enclosing said growth factor therein.

3. The material as defined in claim 1, which is prepared in the form of allowing in-vivo administration by injection and in-vivo gelation after said administration.

## Amended claims

### Amended claims under Art. 19.1 PCT

**1.** (Amended) A material for sustained chemical-release in vivo, comprising:
a hydroxyapatite/collagen (HAp/Col) nanocomposite formed as a coprecipitate; and
a chemical consisting at least one of a drug and a bioactive substance including a growth factor and a DNA enzyme,
wherein said hydroxyapatite (HAp) has a specific surface area in the range of 50 to 300 m²/g,
said hydroxyapatite (HAp) and said collagen (Col) are combined in such a manner that the nanocrystals of said hydroxyapatite (HAp) are oriented in the C-axis direction around the fibers of said collagen (Col), and
said chemical is adsorbed and carried on the surface of said hydroxyapatite (HAp), the surface of said collagen (Col) and in a hydrated water of said hydroxyapatite (HAp) and said collagen (Col).

**2.** The material as defined in claim 1, which further includes glycosaminoglycan or hydrogel which has high hydration ability, wherein said glycosaminoglycan or hydrogel is combined with said HAp/Col nanocomposite so that said HAp/Col nanocomposite serves as a bioabsorbable capsule capable of enclosing said growth factor therein.

**3.** The material as defined in claim 1, which is prepared in the form of allowing in-vivo administration by injection and in-vivo gelation after said administration.
